# EUROPÄISCHE PATENTSCHRIFT

(11) **EP 3 033 073 B1**
(45) Veröffentlichungstag und Bekanntmachung des Hinweises auf die Patenterteilung: **17.05.2017**
(21) Anmeldenummer: 14750493.0
(22) Anmeldetag: 13.08.2014
(51) Int. Cl.: A61K 9/00, A61Q 19/08, A61K 47/10, A61K 47/32, A61K 47/36, A61K 9/10, A61K 36/00, A61K 36/28, A61K 36/31, A61K 36/45, A61K 36/05, A61K 36/18, A61K 8/97, A61K 47/14

(54) **ZUBEREITUNG ZUM SCHUTZ VOR EXTRINSISCHER UND INTRINSISCHER HAUTALTERUNG**
PREPARATION FOR PROTECTION AGAINST EXTRINSIC AND INTRINSIC SKIN AGEING
PRÉPARATION POUR PROTÉGER CONTRE LE VIEILLISSEMENT DE PEAU

(30) Priorität: 16.08.2013 DE 102013216295; 07.08.2014 US 201414453822
(43) Veröffentlichungstag der Anmeldung: 22.06.2016
(73) Patentinhaber: La Prairie Group AG, 8604 Volketswil (CH)
(72) Erfinder: DUDLER, Bernhard, CH-8340 Hinwil (CH); STANGL, Daniel, CH-6045 Meggen (CH)
(74) Vertreter: Hartmann, Jost
(86) Internationale Anmeldenummer: PCT/EP2014/067298
(87) Internationale Veröffentlichungsnummer: WO 2015/022348

(56) Entgegenhaltungen:
- EP-A2- 2 260 829
- JP-A- 2006 104 117
- JP-A- 2009 185 007
- KR-A- 20130 053 151

## Beschreibung

Die vorliegende Erfindung betrifft eine einen oder mehrere Extrakte von Saxifraga oppositifolia (Gegenblättriger Steinbrech) und/oder Soldanella alpina (Alpen-Soldanelle) umfassende kosmetische oder dermatologische Zubereitung sowie deren Verwendung gegen extrinsische und intrinsische Hautalterung.

Als Hautalterung wird der komplexe biologische Prozess der mit dem Alter einhergehenden Veränderung der Haut bezeichnet. Hierbei unterscheidet man die intrinsische Hautalterung, bedingt durch interne physiologische und genetische Faktoren, von der extrinsischen Hautalterung.

Extrinsische Hautalterung ist auf externe Faktoren wie z. B. Umweltfaktoren wie UV-Licht, chemische Reagentien, mechanische Belastung, Zigarettenrauch, Stress oder Luftverschmutzung zurückzuführen. Da UV-Strahlung die Hauptursache für die extrinsische Hautalterung darstellt, spricht man auch von "Photoaging".

Die extrinsischen Faktoren führen beispielsweise zu Faltenbildung, Hauterschlaffung, Verlust von Elastizität und trockenem Aussehen der Haut.

Die intrinsische Hautalterung, auch chronologische Hautalterung genannt, wird durch interne physiologische und genetische Faktoren verursacht und spiegelt Abbauprozesse in der Haut wider. Diese Prozesse sind hauptsächlich auf eine reduzierte Proliferationsaktivität der Hautzellen, eine reduzierte Synthese der Matrixproteine und eine Zunahme der Expression von matrixabbauenden Enzymen zurückzuführen.

Gealterte Zellen zeigen einen Widerstand gegen apoptotische Signale, der zur Akkumulation im Gewebe von nicht proliferierenden gealterten Zellen mit verändertem Genexpressionsmuster führt.

Bei der Hautalterung kommt es häufig zur Bildung von Fältchen und Linien und zum Verlust von Elastizität und Spannkraft.

Hautalterung und Faltenbildung können maßgeblich durch entsprechenden Hautschutz verzögert werden. Im Stand der Technik sind hierzu vielerlei Möglichkeiten dargestellt, wie beispielsweise vom gesundem Lebenswandel bis hin zu topisch applizierbaren kosmetischen und dermatologischen Zubereitungen.

Saxifraga oppositifolia ist eine Pflanze, die bevorzugt in den gemäßigten Breiten auf offenen steinigen Rasen und Moränen in Höhenlagen zwischen 1.600 und 4.500 Meter zu finden ist und welche somit die höchstgelegene Blütenpflanze in Europa darstellt.

Die immergrünen Blätter ertragen Temperaturen bis -40 °C ohne Schaden. Die in kleinen, grundständigen Rosetten gegenständig stehenden Laubblätter sind verkehrt eiförmig bis länglich lanzettlich und 2,5 bis 5 mm lang.

In US 6406682 B1 wird die Pflanzengattung Saxifraga als Bestandteil in oder als Selbstbräunungszubereitung beschrieben.

Die EP 1104670 A2 beschreibt folienartige kosmetische Packungen, die ein wasserlösliches Polymer, einen Saxifraga-Extrakt und Wasser als Lösungsmittel umfasst. Der hierin verwendete Saxifraga-Extrakt wird durch Extrahieren des ungehackten Blattes von Saxifraga (Saxifraga stolonifera Meerb.) mit gereinigtem Wasser, Ethanol, 1,3-Butylenglycol oder dergleichen hergestellt und ist ein Extrakt, der durch Konzentrieren der extrahierten Lösung oder eines durch Trocknen der Extraktlösung und Pulverisieren des getrockneten Extraktes hergestellten Pulvers hergestellt wird.

Soldanella alpina ist eine Pflanze der Primelgewächse. Bevorzugter Standort ist der Halbschatten auf feuchten Böden. Die Blätter sind rundlich und immergrün.

In US 2009/0104295 A1 wird Soldanella Alpina als eine mögliche Quelle einer Wirkstoffgruppe als Zusatz zum Basishaarwuchskomplex aufgeführt. JP2009185007 und JP2006104117 offenbaren die Verwendung von Saxifraga / Saxifraga stolonifera-Extrakten. Es ist wünschenswert, Zubereitungen zur Verfügung zu stellen, die gegen extrinsische und intrinsische Hautalterung wirksam sind.

Die vorliegende Erfindung stellt eine kosmetische oder dermatologische Zubereitung bereit, die (i) einen oder mehrere Extrakte von Saxifraga oppositifolia (Gegenblättriger Steinbrech) und/oder (ii) einen oder mehrere Extrakte von Soldanella alpina (Alpen-Soldanelle) umfasst. Vorzugsweise sind mindestens jeweils mindestens ein Extrakt von Saxifraga oppositifolia und Soldanella alpina in der Zubereitung enthalten.

Besonders bevorzugt ist es, dass die Zubereitung jeweils nur einen Extrakt von Saxifraga oppositifolia und Soldanella alpina umfasst, insbesondere nur diese beiden Extrakte und keine weiteren Pflanzenextrakte.

In gleicher Weise bevorzugt ist es, dass die Zubereitung entweder nur einen oder mehrere Extrakte von Saxifraga oppositifolia oder nur einen oder mehrere Extrakte von Soldanella alpina umfasst. Insbesondere umfasst die Zubereitung dann nur diese einen oder mehrere Extrakte und keine weiteren Pflanzenextrakte.

Bevorzugt wird weiterhin, dass die erfindungsgemäßen Zubereitung zusätzlich einen oder mehrere Extrakte der Gattung Chlamydomonas (auch Chlamydocapsa sp. (Schneealge) genannt) umfasst.

Die gesamte Offenbarung der WO 2012/069073 A1 wird durch Bezugnahme ausdrücklich hierin aufgenommen.

Vorteilhaft umfasst die erfindungsgemäße Zubereitung jeweils einen oder mehrere Extrakte von Saxifraga oppositifolia, Soldanella alpina und Chlamydomonas sp., so dass jeweils einer oder mehrere Extrakte alle drei Pflanzen in der erfindungsgemäßen Zubereitung enthalten sind und durch ihre Hautschutzwirkungen den extrinsischen und intrinsischen Hautalterungsprozess verzögern oder sogar stoppen können.

Weitere Pflanzenextrakte sind bevorzugt dann nicht in den erfindungsgemäßen Zubereitungen enthalten, so dass nur ein oder mehrere Pflanzenextrakte von Saxifraga oppositifolia, Soldanella alpina und der Gattung Chlamydomonas enthalten sind.

Bei den in der erfindungsgemaessen Zubereitung enthaltenen Extrakten kann es sich sowohl um Öl- als auch wasserlösliche Extrakte der Saxifraga oppositifolia und Soldanella alpina handeln. Derartige Extrakte lassen sich beispielsweise nach den folgenden oder ähnlichen Verfahren herstellen.

### Pflanzenmaterial:

Die Biomasse der Pflanzen wird durch den Anbau dieser Pflanzen in Gewächshäusern oder im Freien erhalten. Die Pflanzen können entweder durch Samen (Soldanella) angebaut oder durch Setzlinge multipliziert (Saxifraga) werden, indem sie in angemessene Böden eingepflanzt werden. Sobald erwachsene Pflanzen verfügbar sind, können Mutterpflanzen in kleinere Töchterpflanzen geteilt werden. Das Pflanzenmaterial wird als Biomasse maximal im Frühsommer geerntet. Um einen nachhaltigen Anbau zu gewährleisten und die Zerstörung der Pflanzen zu vermeiden, werden vorzugsweise nur oberirdische Pflanzenteile und besonders bevorzugt nur Zweige (Stiele) und/oder Blätter der Pflanzen verwertet, obwohl beispielsweise Blüten ebenfalls (insbesondere zusätzlich) ebenfalls Verwendung finden können.

Die oberirdischen Pflanzenteile werden geschnitten und danach möglichst umgehend getrocknet, beispielsweise in einem geeigneten Ofen mittels warmer Umluft oder in einem Vakuum-Trockenschrank. Die Trockentemperatur liegt vorzugsweise zwischen 40 und 45 °C, um die temperaturempfindlichen Naturprodukte thermisch möglichst wenig zu belasten.

Durch die Trocknung wird der Feuchigkeitsgehalt des Materials auf vorzugsweise weniger als 10 % reduziert. Danach wird das getrocknete Material beispielsweise in einem Schredder geschnitten, um seine Größe auf vorzugsweise einige wenige Millimeter zu reduzieren. Das Material sollte dann bis zur Weiterverarbeitung an einem kühlen und trockenen Ort gelagert werden.

### Öllösliche Produkte:

Die Herstellung öllöslicher Extrakte kann beispielsweise mittels überkritischer Extraktion mit Kohlendioxid (Supercritical Fluid Extraction, SFE) erfolgen. Hierbei werden mittels SFE mit Kohlendioxid öllösliche Wirkstoffe aus dem getrockneten Pflanzenmaterial extrahiert. Der Extraktionsbehälter wird mit dem getrockneten Pflanzenmaterial gefüllt und das Material wird verdichtet, um Luftkanäle zu beseitigen. Der Druck des Gases beträgt beispielsweise bis etwa 200 bar bei einer Temperatur von etwa 40 °C. Unter diesen Bedingungen wird die dynamische Extraktion üblicherweise etwa 60 Minuten lang durchgeführt. Danach wird das überkritische Kohlendioxid entspannt und rückgeführt und der lipophile Extrakt wird in einem Sammelbehälter gesammelt. Die Extrakte mehrerer Läufe werden vereinigt und beispielsweise in einer entsprechenden Menge an pfanzlichem Öl (z.B. Jojobaöl) mittels Rühren für eine Stunde bei etwa 50 °C gelöst. Nach dem Abkühlen wird die Lösung durch einen 25 µm Filter und einen 4 µm Filter geleitet. Die filtrierte Lösung entspricht dem öllöslichen Extraktprodukt. Dadurch ergeben 1500 g getrocknete Saxifraga beispielsweise etwa 15 kg Produkt (die Ausbeute der Extraktion beträgt üblicherweise 5-10 %), während 900 g getrocknete Soldanella etwa 15 kg Produkt ergeben (die Ausbeute der Extraktion beträgt üblicherweise 2-5 %).

Das extrahierte Pflanzenmaterial kann gesammelt und beispielsweise für die im Folgenden beschriebene hydro-alkoholischen Extraktion verwendet werden.

### Wasserlösliche Produkte:

### 1. Hydro-ethanolische Extraktion

Das Pflanzenmaterial wird mit einer ausreichenden Menge an Wasser und Ethanol (1:1 w/w) vermischt. Die Aufschlämmung wird etwa 24 Stunden bei Raumtemperatur gerührt und danach durch ein 1 µm Filter geleitet. Das solvatisierte Pflanzenmaterial kann ausgepresst werden, um weiteren Extrakt zu gewinnen. Das Filtrat wird mit etwa dem 1,5-fachen Gewicht an Wasser verdünnt und dann bei 4 °C über mehrere Tage hinweg gelagert. Danach wird das Produkt durch einen 4 µm Filter und einen 0,6 µm Filter geleitet und anschliessend einer Sprühgranulierung unterzogen.

### 2. Sprühgranulierung

Die Extraktionslösung wird vor der Sprühgranulierung mit Maltodextrin versetzt. Beispielsweise werden mit 10 kg des getrockneten (<10% Wassergehalt) und geschnittenem Saxifraga-Rohstoffs etwa 100 kg Sprühgranulat und mit 6 kg des getrockneten und geschnittenen Soldanella-Rohstoffs etwa 100 kg Sprühgranulat hergestellt.

Die mit Maltodextrin versetzte Lösung kann in einer Wirbelschicht-Granuliervorrichtung sprühgranuliert werden, beispielsweise under den folgenden Bedingungen:
Granulierung:
   einströmende Luft: 80 °C / 250 bis 800 m³ / h
   Sprührate: 3 bar / 150 - 200 ml
   austretende Luft: 30-50 °C
Trocknung:
   einströmende Luft: 80 °C / 250 bis 800 m³ / h / 5 - 10 Minuten
   austretende Luft: 55 °C
Zeitraum: mehrere Stunden, je nach Chargengröße.

Die obigen lediglich beispielhaften Herstellungsverfahren stellen die bevorzugten Formen der Bereitstellung der erfindungsgemäßen wasser- bzw. öllöslichen Extrakte der Saxifraga oppositifolia und Soldanella alpina dar. Wie oben erwähnt werden dabei vorteilhaft insbesondere nur die Blätter und Pflanzenstiele, nicht aber Blüten und insbesondere nicht die Wurzeln der Pflanzen genutzt.

Da die Extrakte sowohl in öl- als auch in wasserlöslicher Form hergestellt und eingesetzt werden können, ist eine große Freiheit für die Formulierung kosmetischer und pharmazeutischer Präparate gegeben.

Die Extrakte lassen sich in beliebiger Konzentration in kosmetischen oder dermatologischen Zubereitungen einsetzten.

Bevorzugt beträgt die Konzentration von einem oder mehreren Extrakten einer Pflanze (Saxifraga oppositifolia, Soldanella alpina und jeder anderen Pflanze, deren Extrakt(e) gegebenenfalls in der Zubereitung enthalten ist bzw. sind, wie beispielsweise von Chlamydocapsa sp.) in der Zubereitung jeweils unabhängig nicht mehr als 15 Gew.%, beispielsweise nicht mehr als 5 Gew.%, nicht mehr als 1 Gew.%, nicht mehr als 0,5 Gew.%, nicht mehr als 0,1 Gew.%, oder nicht mehr als 0,01 Gew.%, aber vorzugsweise mindestens 0,0001 Gew.%, beispielsweise mindestens 0,0005 Gew.%, mindestens 0,001 Gew.%, oder mindestens 0,002 Gew.%. Üblicherweise beträgt der entsprechende Konzentrationsbereich für den/die Extrakt(e) einer jeden Pflanze 0,0001 bis 0,5 Gew.%, jeweils bezogen auf die Gesamtmasse der Zubereitung.

Die obigen Konzentrationsangaben der erfindungsgemäß eingesetzten Extrakte beziehen sich auf die Masse des reinen (in der Regel pulverförmigen) Extrakts, ohne Lösemittel oder Extraktionsmittel.

Die erfindungsgemäß eingesetzten Extrakte werden beispielsweise in Form der Produkte AlpinEffect Saxifraga SFE OS (0,2 Gew.% Trockenextrakt, Rest Jojobaöl), AlpinEffect Saxifraga WS (0,3 Gew.% Trockenextrakt, > 91 Gew. % Maltodextrin, Rest vornehmlich Wasser), AlpinEffect Soldanella SFE OS (0,08 Gew.% Trockenextrakt, Rest Jojobaöl) und AlpinEffect Soldanella WS (0,4 Gew.% Trockenextrakt, > 91 Gew. % Maltodextrin, Rest vornehmlich Wasser) von der Firma Mibelle (Schweiz) hergestellt.

Werden beispielsweise 1 Gew.% an AlpinEffect Soldanella WS in der Zubereitung eingesetzt, so beträgt der Anteil an tatsächlichem Soldanella alpina Extrakt in der Zubereitung etwa 0,004 Gew.%, bezogen auf die Gesamtmasse der Zubereitung.

Bevorzugt umfassen die erfindungsgemäßen Zubereitungen mindestens zwei der Pflanzenextrakte gewählt aus der Gruppe (i) Saxifraga oppositifolia, (ii) Soldanella alpina und und (iii) Chlamydocapsa sp..

Erfindungsgemäß bevorzugte Zubereitungen umfassen damit
- ein Extrakt aus (i) Saxifraga oppositifolia und ein Extrakt aus (ii) Soldanella alpina,
- ein Extrakt aus (i) Saxifraga oppositifolia und ein Extrakt aus (iii) Chlamydocapsa sp.,
- ein Extrakt aus (ii) Soldanella alpina und ein Extrakt aus (iii) Chlamydocapsa sp. oder
- ein Extrakt aus (i) Saxifraga oppositifolia, ein Extrakt aus (ii) Soldanella alpina und ein Extrakt aus (iii) Chlamydocapsa sp..

Die erfindungsgemäßen Zubereitungen können in den bekannten Formen und Arten vorliegen.

Bevorzugt handelt es sich um eine topische Zubereitung.

Eine Form der Zubereitungen ist als leave-on Zubereitung bekannt, wie beispielsweise als Creme, Lotion oder Körpermilch. Häufig werden diese Zubereitungen als Emulsionen, insbesondere W/O-, O/W-, O/W/O- oder W/O/W-Emulsionen, formuliert. Ebenso können die Zubereitungen Mikroemulsionen, Dispersionen, Gele, wässrige oder alkoholische Lösungen, Seren, Öle, Tuchtränkungsmedien, Tinkturen oder Salben darstellen. Die Extrakte lassen sich vorteilhaft auch appliziert auf oder integriert in Tüchern, Pflastern, Bandagen, Patches oder Pads auf die Haut auftragen.

Die erfindungsgemäße Zubereitung liegt vorteilhaft in Form einer Emulsion vor.

Die erfindungsgemäßen kosmetischen oder dermatologischen Zubereitungen können gegebenfalls weitere aktive Bestandteile enthalten. Lediglich beispielhaft seien hier genannt weitere Extrakte wie beispielsweise einer oder mehrere Extrakte von Artemisia umbelliformis, Rubus idaeus (Himbeere), Plantago lanceolata (Blattextrakt), Saccharomyces cerevisiae, Panax ginseng-Wurzel, Equisetum arvense, und Evernia furfuracea (Baummoos). Weitere Beispiele bevorzugter zusätzlicher aktiver Bestandteile schliessen Biopolymere, Glycoproteine, Vitamine und Antioxidantien ein.

Die erfindungsgemäßen kosmetischen oder dermatologischen Zubereitungen können ferner Hilfsstoffe und weitere Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, Selbstbräuner, Puffer, pH Regulatoren, Tenside, Treibgase, Puder, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitraspirantien, Bleich und Färbemittel etc, sofern der Zusatz die geforderten Eigenschaften hinsichtlich geforderter Stabilität, Sensorik und vor allem hinsichtlich der Schutzfunktion sowie Haut- und Haarverträglichkeit nicht signifikant beeinträchtigt oder ausgeschlossen werden.

Bevorzugt umfassen die erfindungsgemäßen Zubereitungen zusätzlich ein oder mehrere Hautbefeuchtungsmittel.
Auch der Zusatz an einem oder mehreren Lichtschutzfiltern ist vorteilhaft.

Erfindungsgemäß können einer oder mehrere Extrakte von Saxifraga oppositifolia (Gegenblättriger Steinbrech) und/oder Soldanella alpina (Alpen-Soldanelle) in kosmetischen oder dermatologischen Zubereitungen zur Verminderung oder Vermeidung von Hautschäden durch extrinsische und/oder intrinsischen Faktoren verwendet werden bzw. können zur Herstellung pharmazeutischer, insbesondere dermatologischer Zubereitungen verwendet werden und zur Verminderung oder Vermeidung von Hautschäden durch extrinsische und/oder intrinsischen Faktoren dienen.

Vorteilhaft ist die Nicht-therapeutische Verwendung von kosmetischen Zubereitungen umfassend (i) ein oder mehrere Extrakte von Saxifraga oppositifolia und/oder (ii) ein oder mehrere Extrakte von Soldanella alpina sowie gegebenenfalls einen oder mehrere Extrakte von Chlamydocapsa sp. zur Verminderung oder Vermeidung von Hautschäden durch extrinsische und/oder intrinsischen Faktoren.

Bevorzugt umfasst die Zubereitung dabei mindestens zwei der Extrakte.

Ein Verfahren zur Verminderung oder Vermeidung von Hautschäden durch extrinsische und/oder intrinsischen Faktoren, welches die topische Applikation einer erfindungsgemäßen Zubereitung auf die Haut umfasst ist ebenfalls erfindungsgemäß.

Desgleichen ein Verfahren zur Verzögerung des intrinsischen und/oder extrinsischen Hautalterung, welches die topische Applikation einer erfindungsgemäßen Zubereitung auf die Haut umfasst, erfindungsgemäß.

Die erfindungsgemäßen Zubereitungen helfen der Haut sich vor den sichtbaren Zeichen vorzeitiger Hautalterung zu schützen, UV - Stress zu mildern, Falten zu glätten, die Feuchtigkeitsbarriere wieder herzustellen und dadurch die Haut widerstandsfähiger zu machen im Vergleich zur Haut ohne die erfindungsgemäße Anwendung.

Die erfindungsgemäßen Extrakte sind bevorzugt in topisch applizierbaren Zubereitungen enthalten. Insbesondere ist die topisch applizierbare Zubereitung eine kosmetische Zubereitung.

Einer oder mehrere Extrakte von Saxifraga oppositifolia und/oder Soldanella alpina lassen sich somit auch zur Herstellung pharmazeutischer, insbesondere dermatologischer Zubereitungen verwenden.

Bevorzugt werden die erfindungsgemäßen Extrakte in Kombination mit einem oder mehreren Extrakten von Chlamydocapsa sp. (Schneealge) verwendet.

In einer weiteren Ausführungsform umfassen die erfindungsgemäßen Zubereitung zusätzlich einen oder mehrere aus den folgenden Extrakten ausgewählte Extrakte: Artemisia umbelliformis Extrakt, Rubus idaeus (Himbeer) Extrakt, Plantago lanceolata Blattextrakt, Saccharomyces cerevisiae Extrakt, Panax ginseng Wurzelextrakt, Equisetum arvense Extrakt, Evernia furfuracea (Baummoos) Extrakt.

Die Extraktion kann dabei nach bekannten Verfahren und unter Zuhilfenahme bekannter Extraktionshilfsmittel durchgeführt werden. In der Extraktionstechnologie können verschiedene Verfahren angewandt werden, z. B. Mazeration, Dimazeration, Digestion, Re-/Perkolation, Soxhletverfahren, Extraktion nach Twisselmann, Turbo- (Wirbel), Ultra-Turrax, Ultraschall-, Gegenstrom-Extraktion und Extraktion mittels Zentrifugalextraktor.

Weiterhin kann die erfindungsgemäße Zubereitung zusätzlich mindestens eine Aminosäure umfassen.

Im Folgenden werden die vorteilhaften Eigenschaften der erfindungsgemässen Zubereitung anhand der Ergebnisse von verschiedenen Tests veranschaulicht. In den Tests wurden die oben erwähnten wasserlöslichen Extraktprodukte AlpinEffect Saxifraga WS und AlpinEffect Soldanella WS exemplarisch für die erfindungsgemäßen Extrakte eingesetzt. Die Prozentangaben beziehen sich auf tatsächlich eingesetzten Extrakt (d.h. ohne die in diesen Produkten enthaltenen Zusätze wie beispielsweise Maltodextrin).

### 1. Mitochondriale Funktion unter oxidativem Stress

Bekannt ist, dass Mitochondrien für die Energieerzeugung in menschlichen Zellen zuständig sind. Sie befinden sich im Zytoplasma und dienen den Zellen als "Batterien", um Energie zu produzieren, zu speichern und zu verteilen. Die menschliche Zelle enthält durchschnittlich 1500 Mitochondrien. Zellen mit hoher Stoffwechselleistung (z.B. Muskeln oder die Leber) enthalten mehr Mitochondrien. Die Mitochondrien bewegen sich im Zytoplasma je nach Bedarf der Zelle. Sie sind mit Ihrer eigenen DNA ausgestattet und können sich deswegen unabhängig von der Teilung der Zelle eigenständig vermehren. Ohne die Mitochondrien ist die Zelle funktionsunfähig und kein Leben ist möglich. Wenn diese Kraftwerke der Zellen nicht fehlerfrei arbeiten, kann dies Alterungsprozesse in der Haut beschleunigen. Defekte in den Mitochondrien, insbesondere auch in der mitochondrialen DNA, können daher die Alterung beschleunigen.

Der Schutz der mitochondrialen Funktionalität vor extrinsischen Störfaktoren, z.B. vor UV-Strahlung und oxidativem Stress, bzw. die Gewährleistung der Integrität der Mitochondrien ist daher ein wirksamer Schutz vor der Hautalterung.

Der folgende Test zeigt, dass 10 mM Wasserstoffperoxidlösung das Membranpotential der Mitochondrien als Mass für gesunde, metabolisch aktive Hautzellen um ca. 50 % reduziert. Wasserstoffperoxid ist somit beispielhaft für extrinsische Störfaktoren.

In Gegenwart von sowohl des Saxifraga- als auch des Soldanella-Extraktes beträgt die Reduktion des Membranpotentials durch Wasserstoffperoxid nur ca. 25 %, d.h. die erfindungsgemäßen Extrakte schützen die Hautzellen wirksam vor oxidativem Stress und damit vor extrinsischer Hautalterung.

Bei der Durchführung des Tests wurden primäre humane epidermale Keratinozyten, genauer "transit amplifying cells", verwendet.

Um den Einfluss des Extraktes auf mitochondriale Funktionalität nach einer oxidativen Belastung zu analysieren, wurde ein JC-1 Mitochondrienmembranpotential-Assay durchgeführt. In gesunden Zellen ist das metabolisch aktive mitochondriale Potential hoch und der Farbstoff JC-1 bildet spontan Komplexe (J-Aggregate) und zeigt dadurch eine intensiv rote Fluoreszenz. Andererseits verbleibt der Farbstoff JC-1 bei geschädigten Zellen mit niedrigem Membranpotential in der unkomplexierten, monomeren Form. Das Verhältnis der Fluoreszenzintensität von J-Aggregaten zu JC-1 Monomeren dient als Mass für die Funktionalität der Zelle.

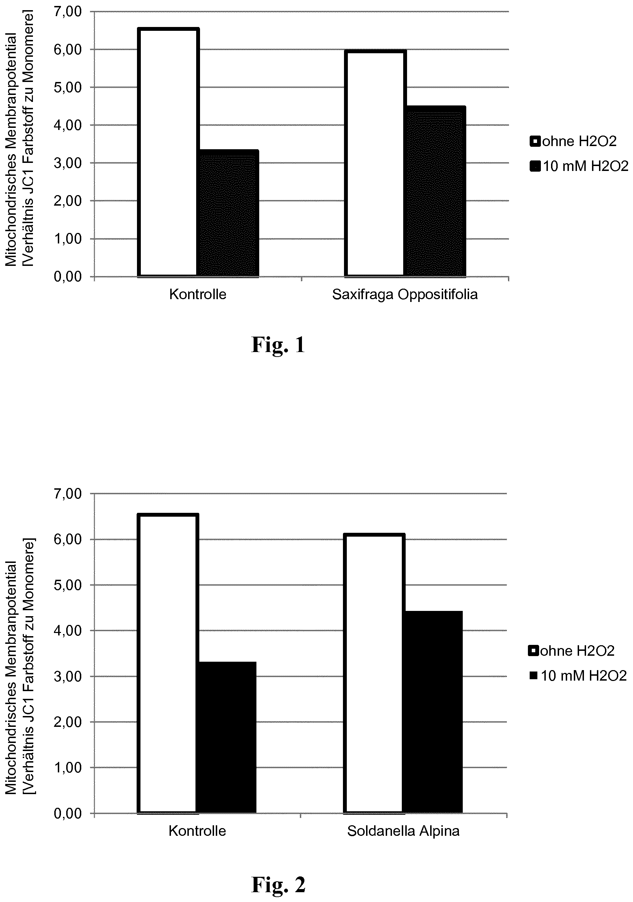

In den Figuren 1 und 2 sind die Testergebnisse graphisch dargestellt. Diese Figuren zeigen die Wirksamkeit und Schutzfunktion der erfindungsgemäßen Extrakte gegen extrinsische Störfaktoren.

Die im Test eingesetzte Konzentration von Saxifraga oppositifolia betrug 0,00024 Gew.% und diejenige von Soldanella alpina betrug 0,00024 Gew.% (bezogen auf das Gewicht des Zellmediums).

### 2. Schutz mitochondrialer DNA bei UV-Bestrahlung

Der folgende Assay zeigt die Schutzwirkung der erfindungsgemäßen Extrakte bezüglich mitochondrialer DNA bei UV-Bestrahlung.

Auf der mitochondrialen DNA (mtDNA) befinden sich einige, wenn auch nicht alle, Gene für die Enzyme der Atmungskette, sowie Gene, die für die Struktur und Reproduktion der Mitochondrien verantwortlich sind. Schäden können an der mtDNA sehr leicht auftreten, liegt diese doch ungeschützt in den Mitochondrien vor und ist dort den freien Radikalen, die während der Energieproduktion anfallen können, ausgesetzt. Schäden an der mtDNA können daher zu einer starken Beeinträchtigung der zellulären Energiegewinnung führen.

Einer der häufigsten Schädigungen der mtDNA, die im nachfolgenden Assay detektiert wird, wird als "common deletion" bezeichnet.

HaCaT Zellen sind Zellen einer spezifischen humanen Keratinozyten-Zelllinie. Kultivierte Keratinozyten (HaCaT) werden für 48 h mit verschiedenen Verbindungen inkubiert und anschließend für 1 h mit UVB Strahlung (1,5 mJ/cm²) gestresst. Die Zellen werden dann gesammelt und zwecks DNA-Extraktion lysiert. Mit Hilfe der Intensität der common deletion-Bande, ausgedrückt als Verhältnis der common deletion gegenüber Standard, kann der Schutz gegen Schädigung durch UV-Licht bestimmt werden.

Untersucht wurden folgende Verbindungen:

| Pflanzenextrakte | Anteil Gew.% | % Reduktion der durch UVB induzierten DNA-Schäden (Common Deletion Test) | Figur |
|---|---|---|---|
| Saxifraga oppositifolia | 0,002% | - 46% | 3a |
| Soldanella alpina | 0,002% | - 52% | 3a |
| Saxifraga + Soldanella | Saxifraga = 0,0003% | - 100% | 3b |
| | Soldanella = 0,00036% | | |
| Saxifraga + Soldanella + Schneealgen | Saxifraga = 0,00033% | - 92% | 3c |
| | Soldanella = 0,0004% | | |
| | Schneealgen = 0,0022% | | |

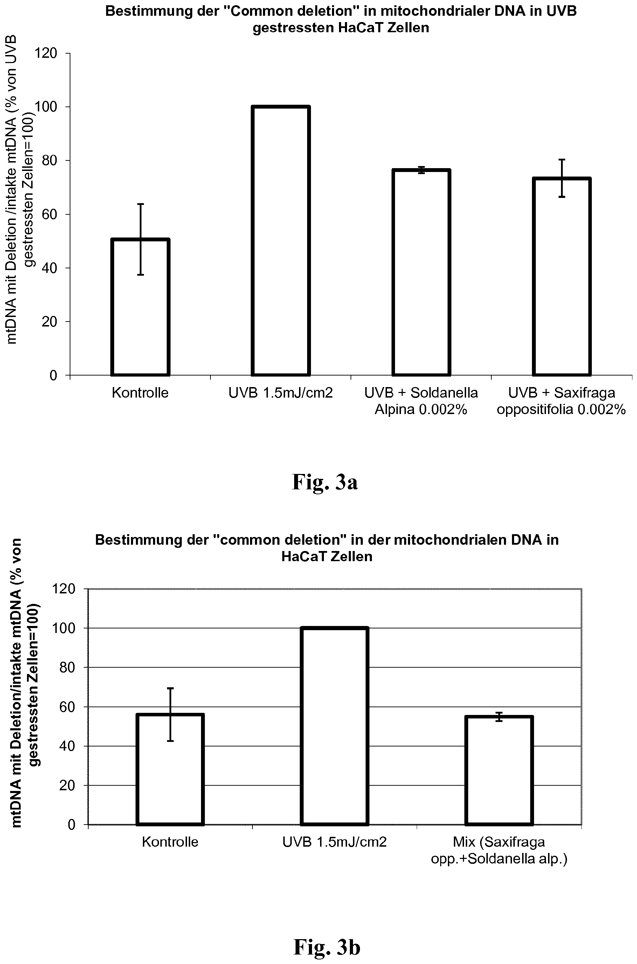

Figur 3a zeigt, dass Soldanella alpina Extrakt die UV-induzierten Schäden der mitochondrialen DNA in Keratinozyten (HaCaT-Zellen) um 52 % und Saxifraga oppositifolia Extrakt um 46 % relativ zur Kontrolle reduziert.

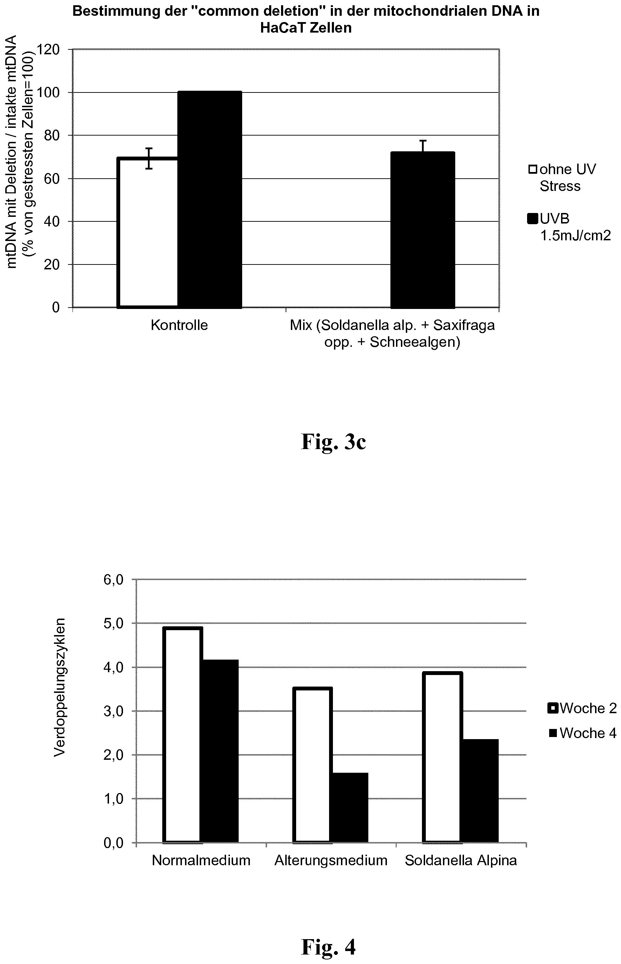

Die Kombination der Extrakten von Saxifraga oppositifolia und Soldanella alpina reduziert die UV-induzierten Schäden um 100 % (Abbildung 3b). Selbst bei einem Anteil von 0,0003 % - 0,00036 % an erfindungsgemäß verwendeten Extrakten wird ein signifikanter Schutz beobachtet, der wie Figur 3 b zeigt, bei Konzentrationserhöhung und Kombination der Extrakte auf 100 % erhöht werden kann.

Auch die Kombination mit einem weiteren bevorzugt eingesetzten Algen- bzw. Pflanzenextrakt, Chlamydomonas (Chlamydocapsa sp. (Schneealge)), führt zu einem nahezu 100 %-igen Schutz gegenüber durch UV-Strahlung induzierten Schäden (Figur 3c).

### 3. Schutz zellulärer DNA bei UV-Bestrahlung

DNA-Schäden führen zu einer Beeinträchtigung zellulärer Funktionen und letztendlich zu Hautalterung. UV-Strahlung ist der wesentliche Faktor bei vorzeitiger (extrinsischer) Hautalterung.

3D epidermale Kulturen wurden unter Verwendung primärer humaner Keratinozyten und 3D Prime Culture-Medium untersucht. Nach 18 Tagen wurden die Kulturen für 1 Stunde mit 100 µl entweder einer Lösung von Soldanella alpina Extrakt in Medium oder Medium allein (unbehandelte Kontrollen) behandelt. Bei einer Bestrahlung von 1200 mJ/cm² (gesamt UV) wurden die Proben dann vier Stunden UVB / UVA-Licht (20 % / 80 %) ausgesetzt. Dann wurden die Proben fixiert, in Paraffin eingebettet, geschnitten und unter Verwendung eines monoklonalen Antikörpers mit Thymindimeren gefärbt. Anschließend wurden mit einem Fluoreszenzmikroskop Bilder aufgenommen.

### Resultat:

In der unbehandelten Kontrolle wurden keine Thymindimere beobachtet. In UV-exponierten Kontrollen wurden nukleare Thymindimere in den unteren Schichten der Proben deutlich sichtbar. Im Gegensatz dazu waren in den mit Soldanella alpina (0,1 %) und UV-behandelten Zellen keine Dimere sichtbar. Dies zeigt, dass der Extrakt aus Soldanella alpina die Bildung von UV-induzierten DNA-Schäden in den Zellen eines epidermalen dreidimensionalen Hautmodells verhindert und damit der extrinsischen Hautalterung bzw. Photoaging vorbeugt.

### 4. Verzögerung des Alterungsprozesses von humanen Keratinozyten (intrisische Hautalterung)

Neben der Schutzfunktion vor extrinsischen Faktoren zeigen die erfindungsgemäß eingesetzten Extrakte auch eine Wirksamkeit hinsichtlich der Verzögerung des Alterungsprozesses von humanen Keratinozyten (intrinsische Hautalterung).

Um dies zu veranschaulichen, wurden humane Keratinozyten in früher Wachstumsphase (Transit Amplifying Cells) in einem definierten Medium, in dem diese Zellen innerhalb von vier Wochen altern, mit und ohne Soldanella-Extrakt inkubiert und die Proliferationsrate der Zellen wurde als Mass für deren Alterung bestimmt.

Um die intrinsische Anti-Aging-Funktion der Extrakte zu bewerten, wurden die Zellen in CnT-07-Medium (progenitor cell targeted medium) oder in CnT-AG1-Medium, einem vollständig definierten Alterungsmedium, in dem die Keratinozyten über einen Zeitraum von 4 Wochen altern, kultiviert. Die Anzeichen des Alterns umfassen eine Verminderung der Proliferationsrate. Die Proliferation wurde jede Woche mit einem automatisierten Zellzähler gemessen. Während der ersten zwei Wochen des Alterns waren die Proliferationsraten der Keratinozyten, die mit Pflanzenextrakten behandelt wurden, ähnlich denen der im CnT-AG1-Medium allein wachsenden Zellen. Nach 3- bis 4-wöchigem Alterungsprozess erhöhten sich die Unterschiede jedoch. In der 4. Woche war die Proliferationsrate der Zellen mit Soldanella alpina Extrakt (0,00024%) im Vergleich zur CnT-AG1-Kontrolle um über 40 % höher.

Figur 4 zeigt, dass die Proliferation der Zellen im Alterungsmedium in Gegenwart des Soldanella alpina Extraktes nach 2 und 4 Wochen höher ist als im Alterungsmedium ohne Extrakt. Da gealterte Zellen sich nicht mehr so schnell vermehren und somit eine geringere Proliferationsrate als junge Zellen aufweisen, zeigt dies, dass das intrinsische Altern der Zellen durch den Soldanella alpina Extrakt verlangsamt wird.

### 5. Bildung epidermaler Haut nach UV-Bestrahlung

Auch die Neubildung epidermaler Haut wird durch UV-Bestrahlung gestört.

Die Erneuerung der Epidermis wird durch die epidermalen Stammzellen und ihre direkten Nachkommen, den "Transit Amplifying Cells" , sichergestellt. Verlieren diese Zellen ihre Fähigkeit zur Hauterneuerung, sei es durch Altern oder durch externen Stress, z.B. UV-Strahlung, so verlangsamt sich die Hauterneuerung generell und die Qualität, sprich die Dicke der Epidermis, nimmt ab und Zeichen der Hautalterung werden sichtbar.

### Testdesign:

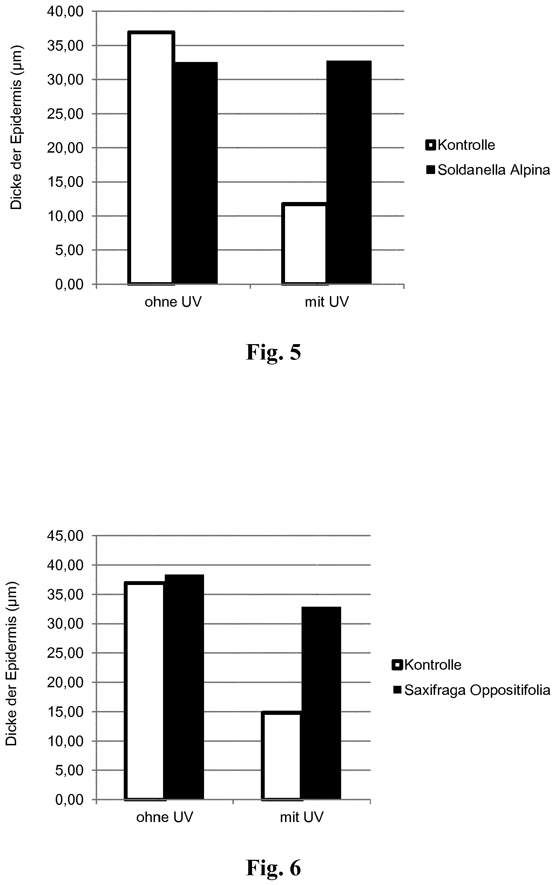

Ein wichtiger Indikator für die Keratinozyten-Progenitorzellen-Funktion ist die Möglichkeit, eine geschichtete, 3D epidermale Struktur zu bilden. Um die Fähigkeit der erfindungsgemäss eingesetzten Pflanzenextrakte, vor dem Verlust der Progenitorzellen-Funktion schützen, zu bewerten, wurden Keratinozyten bis auf 80 % Konfluenz in Kulturmedium wachsen gelassen und dann 24 Stunden lang mit 0,002 % Soldanella alpina Extrakt oder mit 0,002 % Saxifraga oppositifolia Extrakt behandelt. Die Zellen wurden dann UVB / UVA-Licht ausgesetzt. Unbelichtete Proben dienten als negative Kontrolle. Eine Stunde nach der UV-Behandlung wurden die Zellen für den Aufbau von 3D-Modellen unter Verwendung des Standardprotokolls ausgesät. Am 18. Tag der Airlift-Kultur wurden 3D-Modell-Proben genommen, in Formalin eingebettet, in Paraffin fixiert, geschnitten und angefärbt. Histologische Schnitte wurden anschließend unter dem Lichtmikroskop ausgewertet. Es wurden Duplikatbilder aufgenommen und für jeden Zustand wurde die epidermale Dicke (von der basalen Schicht nach unten zur Hornschicht) gemessen. Als Ergebnis wurde festgestellt, dass beide erfindungsgemäß verwendeten Extrakte das Hauterneuerungspotential von humanen Hautzellen unter UV-Stress bewahren. Ohne Vorbehandlung der Hautzellen mit einem der beiden Extrakte verlieren die Zellen ihre Fähigkeit, *in vitro* eine intakte dreidimensionale Epidermis zu bilden, wenn sie UV-Strahlung ausgesetzt werden. Die Extrakte schützen daher die Fähigkeit der "Transit Amplifying Cells" zur Hauterneuerung unter UV-Stress. Die epidermale Dicke der Haut bleibt erhalten, wie in den Figuren 5 und 6 dargestellt.

### 6. Aktivierung des Proteasoms in HaCaT-Zellen nach UV-Stress

In einem weiteren Assay wurde die Aktivierung des Proteasoms in HaCaT-Zellen nach UV-Stress untersucht.

Das Proteasom ist ein Proteinkomplex, der im Cytoplasma und im Zellkern (bei Eukaryoten) defekte und nicht mehr benötigte Proteine zu Fragmenten abbaut. Das Proteasom ist somit ein Bestandteil der Proteinqualitätskontrolle.

UV-Stress hemmt die proteolytische Aktivität des Proteasoms, was zur Akkumulation defekter bzw. nicht mehr benötigter Proteine führt und letztendlich zelluläre Funktionen beeinträchtigt. Falls defekte bzw. oxidierte Proteine aggregieren, kann dies zur Bildung des Alterspigmentes Lipofuszin führen.

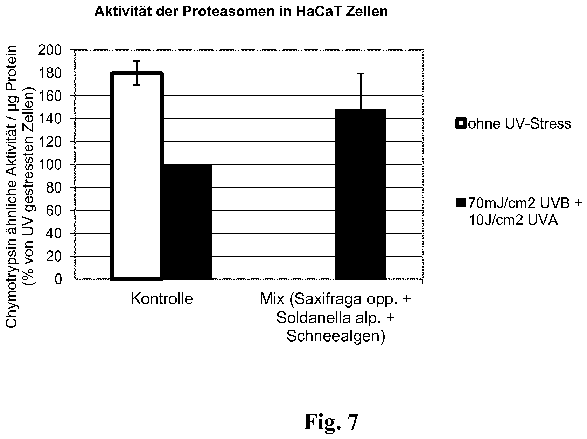

In Figur 7 sind die Resultate nach der Behandlung der Proteasome mit der erfindungsgemäßen Kombination von drei Pflanzenextrakten (Saxifraga oppositifolia, Soldanella alpina und Chlamydocapsa sp.) gezeigt: die Aktivität der Proteasome in HaCaT-Zellen nach UV-Bestrahlung (70 mJ/cm² UVB + 10 J/cm² UVA) wird wieder erhöht bzw. reaktiviert, und zwar um 48%.

Die Konzentrationen (Gew.%) der Extrakte betrugen: Saxifraga 0,00033 %, Soldanella 0,00044 % und Chlamydocapsa 0,0022 %.

### 7. Hemmung der Freisetzung der Matrix- Metalloproteinasen MMP-1 und MMP-3 nach UVA-Stress in humanen dermalen Fibroblasten

Weitere Tests wurden bezüglich der Hemmung der Freisetzung der Matrix-Metalloproteinasen MMP-1 und MMP-3 nach UVA-Stress in humanen dermalen Fibroblasten durchgeführt.

Matrix-Metalloproteinasen sind Proteasen, die Komponenten der extrazellulären Matrix abbauen. UV-Licht kann die Expression von Matrix-Metalloproteinasen stimulieren.

MMP-1 ist eine Kollagenase, d.h. eine Kollagen abbauende Proteinase. MMP-3 baut neben verschiedenen Kollagenen auch Fibronectin, Elastin und Laminin ab und spielt eine zentrale Rolle in der Modulation des Bindegewebes und in der Wundheilung.

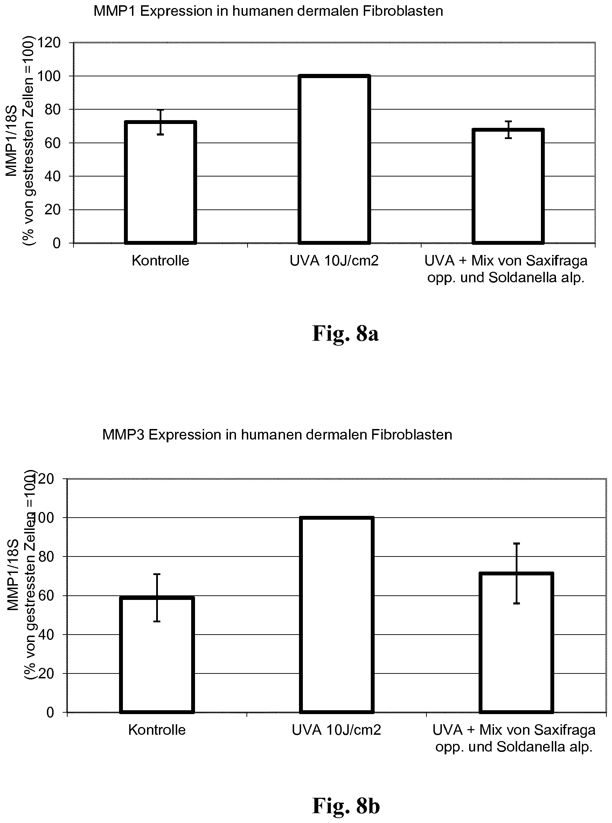

Eine erfindungsgemäße Zubereitung, die eine Kombination von Extrakten von Saxifraga oppositifolia (0,00135 %) und Soldanella alpina (0,0018 %) umfasst, hemmt die UVinduzierte Expression von MMP-1 und MMP-3 signifikant und beugt dadurch der lichtinduzierten Hautalterung vor, wie aus den Figuren 8a und 8b deutlich wird.

### 8. Kolonienbildung durch primäre epidermale Keratinozyten-Vorläuferzellen nach UV-Bestrahlung oder Behandlung mit Wasserstoffperoxid

Die erfindungsgemäße Kombination der Extrakte von Saxifraga oppositifolia, Soldanella alpina und/oder Chlamydomonas sp., insbesondere die Kombination aller drei Extrakte, schützt das Proliferationspotenzial epidermaler Stammzellen unter UV- und oxidativem Stress, wie gemessen an der Fähigkeit der Zellen, Kolonien *in vitro* zu bilden (Colony Forming Efficiency = CFE). Dazu wurden folgende Experimente durchgeführt:
UV Bestrahlung:
   Primäre epidermale Keratinozyten- Vorläuferzellen wurden in Gegenwart der Extraktkombination in CnT-07 Kulturmedium ausgesät und 48 Stunden lang wachsen gelassen. Jede Probe wird dann einer Bestrahlung mit einer UVA-und UVB-Lichtquelle (1200 mJ oder 1800 mJ) ausgesetzt. Ein Test mit nicht bestrahlter Probe wurde ebenfalls durchgeführt. Nach der Belichtung wurden die Zellen für die CFE Assays mit einer niedrigen Dichte ausgesät und gezüchtet. Die Kulturen werden dann fixiert und angefärbt und die Kolonien wurden gezählt. Die CFE Auswertungen wurden dreifach durchgeführt. Unbehandelte Zellen wurden als Kontrolle verwendet.
Wasserstoffperoxid:
   Mit einem ähnlichen experimentellen Design wie für die UV-Bestrahlung beschrieben wurde die CFE bei Peroxidbelastung ausgewertet.

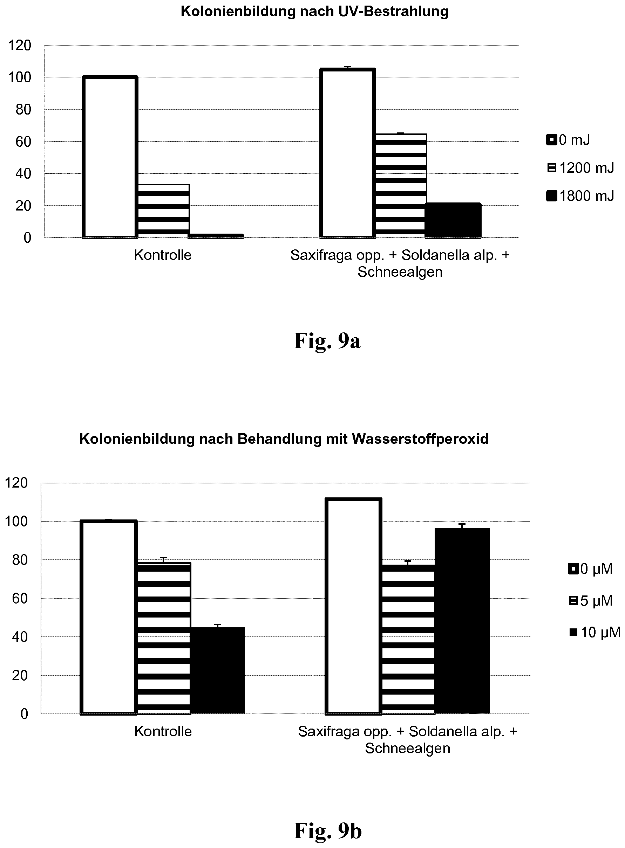

Wie die in Figur 9a graphisch dargestellten Resultate zeigen, schützt die erfindungsgemäss eingesetzte Extraktkombination das Proliferationspotential der epidermalen Stammzellen signifikant vor UV-Strahlung. So verdoppelt sich die Anzahl der gebildeten Kolonien in Gegenwart der Extrakte im Vergleich zur unbehandelten Kontrolle bei 1200 mJ/cm². Desgleichen schützen die Extrakte vor den negativen Einflüssen von Peroxiden (10 µM) auf die Proliferationsfähigkeit der epidermalen Stammzellen (Fig. 9b).

### 9. Bildung sphärischer Kolonien durch dermale Vorläuferzellen nach UV-Bestrahlung

Die erfindungsgemäss eingesetzten Pflanzenextrakte schützen nicht nur epidermale, sondern auch dermale Stammzellen gegen die negativen Auswirkungen von UV-Strahlung auf deren Proliferationsfähigkeit.

Dermale Vorläuferzellen (isoliert aus dermaler Papille) wurden in Monoschichten in Gegenwart oder Abwesenheit der Extrakte für einen Zeitraum von 2 Tagen kultiviert und dann mit einer UVA-und UVB-Lichtquelle mit einer Dosis von 1200 mJ oder 1800 mJ bestrahlt. Die Bildung sphärischer Kolonien wurde nach ca. 5 Tagen der Kultivierung ausgewertet.

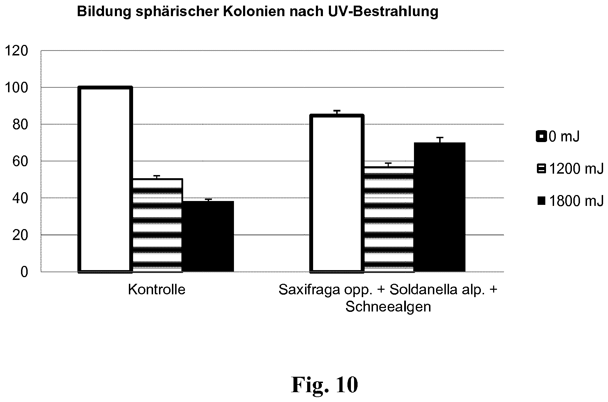

Die Proliferationsfähigkeit der dermalen Stammzellen wurde anhand ihrer Fähigkeit, sphärische Kolonien zu bilden (Sphären), ermittelt. Während in der Kontrolle (ohne Extrakte) die Anzahl der gebildeten Sphären unter UV-Bestrahlung (1200 mJ bzw. 1800 mJ) um 50 % bzw. 62 % sank, stieg die Anzahl der Sphären in Gegenwart der Extrakte um bis zu 100 % (Figur 10).

Epidermale und dermale Stammzellen bzw. deren Nachkommen sind wesentlich am routinemässigen Unterhalt bzw. Erneuerung der entsprechenden Hautschicht beteiligt. Der Schutz dieser Zellen vor externen Stressoren wie UV-Strahlung oder freien Radikalen trägt somit wesentlich zum Erhalt einer jugendlichen, gesunden und schönen Haut bei.

Die folgenden nicht-beschränkenden Beispiele sollen die vorliegende Erfindung weiter veranschaulichen.

### Beispiel 1

**Pflegende Öl-haltige Zubereitung**

| **INCI** | **Gew. %** |
|---|---|
| PRUNUS AMYGDALUS DULCIS (SWEET ALMOND) OIL | 91,0370 |
| HELIANTHUS ANNUUS (SUNFLOWER) SEED OIL | 4,0000 |
| SIMMONDSIA CHINENSIS (JOJOBA) SEED OIL | 1,1000 |
| SILYBUM MARIANUM ETHYL ESTER | 1,0000 |
| FRAGRANCE (PARFUM) | 1,5000 |
| PHENOXYETHANOL | 0,6000 |
| CAPRYLIC/CAPRIC TRIGLYCERIDE | 0,5000 |
| TOCOPHERYL ACETATE | 0,1000 |
| C10-18 TRIGLYCERIDES | 0,0995 |
| BRASSICA CAMPESTRIS (RAPESEED) SEED OIL | 0,0495 |
| ALGAE EXTRACT | 0,0100 |
| SAXIFRAGA OPPOSITIFOLIA EXTRACT | 0,0040 |
| SOLDANELLA ALPINA EXTRACT | 0,0016 |
| | 100,0000 |

Die Formulierung kann vorteilhaft ergänzt werden mit weiteren pflanzlichen Ölen, öllöslichen Vitaminen und/oder Antioxidantien.

### Beispiel 2

**W/O - Emulsion**

| **INCI** | **Gew. %** |
|---|---|
| WATER (AQUA) | 42,400 |
| GLYCERIN | 15,170 |
| CAPRYLIC/CAPRIC TRIGLYCERIDE | 10,500 |
| BUTYLENE GLYCOL | 6,150 |
| COCO-CAPRYLATE/CAPRATE | 4,000 |
| CYCLOPENTASILOXANE | 3,500 |
| DIMETHICONE | 3,000 |
| GLYCERYL STEARATE | 2,000 |
| CETYL ALCOHOL | 2,000 |
| MALTODEXTRIN | 1,900 |
| PEG-40 STEARATE | 1,800 |
| POTASSIUM CETYL PHOSPHATE | 1,600 |
| BUTYROSPERMUM PARKII (SHEA BUTTER) | 1,500 |
| PHENOXYETHANOL | 0,900 |
| SORBITAN TRISTEARATE | 0,700 |
| CARBOMER | 0,500 |
| FRAGRANCE (PARFUM) | 0,350 |
| CALCIUM ALUMINUM BOROSILICATE | 0,338 |
| ETHYLHEXYLGLYCERIN | 0,300 |
| SODIUM ACRYLATE/SODIUM ACRYLOYLDIMETHYL TAURATE COPOLYMER | 0,247 |
| PROPANEDIOL | 0,200 |
| SODIUM HYDROXIDE | 0,176 |
| ISOHEXADECANE | 0,163 |
| PENTYLENE GLYCOL | 0,150 |
| TITANIUM DIOXIDE | 0,135 |
| ISOMALT | 0,092 |
| SODIUM HYALURONATE | 0,070 |
| TRITICUM VULGARE (WHEAT) FLOUR LIPIDS | 0,050 |
| POLYSORBATE 80 | 0,046 |
| PEG-8 | 0,025 |
| SILICA | 0,023 |
| CHLAMYDOMONAS EXTRACT | 0,006 |
| SOLDANELLA ALPINA EXTRACT | 0,004 |
| SAXIFRAGA OPPOSITIFOLIA EXTRACT | 0,003 |
| CITRIC ACID | 0,002 |
| POTASSIUM SORBATE | 0,001 |
| | 100,000 |

Die in den Beispielen eingesetzten öl- und wasserlöslichen Extrakte zeigen beide die erwähnten Vorteile hinsichtlich der Schutzfunktion gegenüber extrinsischen und intrinsischen Einflussfaktoren.

Diese Zubereitung kann vorteilhaft zusätzlich eine oder mehrere der folgende Komponenten enthalten:
Cellular Complex (La Prairie), Peptide, Pflanzenextrakte, Extrakte pflanzlicher Stammzellen, Biopolymere, Vitamine, Antioxidantien.
Der Cellular Complex umfasst eine Kombination aus
   a) Glycoprotein 1, erhältlich aus der gereinigten zytoplasmatischen Fraktion aus Hefen (Saccharomyces),
   b) Glycoprotein 2, erhältlich aus der gereinigten zytoplasmatischen Fraktion aus Lactobacillus,
   c) Ginsengextrakt und
   d) Schachtelhalmextrakt (horsetail extract, Equisetum Arvense extract)
   entsprechend der US 5840309.

## Patentansprüche

1. Kosmetische oder dermatologische Zubereitung umfassend (i) einen oder mehrere Extrakte von Saxifraga oppositifolia und/oder (ii) einen oder mehrere Extrakte von Soldanella alpina.

2. Zubereitung nach Anspruch 1, umfassend zumindest (i) einen oder mehrere Extrakte von Saxifraga oppositifolia.

3. Zubereitung nach Anspruch 1, umfassend zumindest (ii) einen oder mehrere Extrakte von Soldanella alpina.

4. Zubereitung nach Anspruch 1, umfassend sowohl (i) einen oder mehrere Extrakte von Saxifraga oppositifolia als auch (ii) einen oder mehrere Extrakte von Soldanella alpina.

5. Zubereitung nach irgendeinem der vorangehenden Ansprüche, welche bezogen auf die Gesamtmasse der Zubereitung nicht mehr als 15 Gew.%, bevorzugt nicht mehr als 5 Gew.%, und insbesondere von 0,0001 bis 0,5 Gew.% von jeweils (i) und/oder (ii) enthält.

6. Zubereitung nach irgendeinem der vorangehenden Ansprüche, in welcher (i) und/oder (ii) mindestens einen wasserlöslichen Extrakt umfassen.

7. Zubereitung nach irgendeinem der vorangehenden Ansprüche, in welcher (i) und/oder (ii) mindestens einen öllöslichen Extrakt umfassen.

8. Zubereitung nach irgendeinem der vorangehenden Ansprüche, in welcher alle Extrakte (i) und/oder (ii) entweder wasserlöslich oder öllöslich sind.

9. Zubereitung nach irgendeinem der vorangehenden Ansprüche, in welcher (i) und/oder (ii) durch Extraktion von Pfanzenblättern und/oder Pflanzenstielen erhalten wurden.

10. Zubereitung nach irgendeinem der vorangehenden Ansprüche, welche zusätzlich (iii) einen oder mehrere Extrakte von Chlamydocapsa sp. umfasst.

11. Zubereitung nach irgendeinem der vorangehenden Ansprüche, welche zusätzlich mindestens einen von (i), (ii) und (iii) verschiedenen Extrakt umfasst.

12. Zubereitung nach Anspruch 11, welche zusätzlich einen oder mehrere aus den folgenden Extrakten ausgewählte Extrakte umfasst: Artemisia umbelliformis Extrakt, Rubus idaeus (Himbeer) Extrakt, Plantago lanceolata Blattextrakt, Saccharomyces cerevisiae Extrakt, Panax ginseng Wurzelextrakt, Equisetum arvense Extrakt, Evernia furfuracea (Baummoos) Extrakt.

13. Zubereitung nach irgendeinem der vorangehenden Ansprüche 1 bis 10, welche keine zusätzlichen Pflanzenextrakte enthält.

14. Zubereitung nach irgendeinem der vorangehenden Ansprüche, welche zusätzlich mindestens eine Aminosäure umfasst.

15. Zubereitung nach irgendeinem der vorangehenden Ansprüche, welche in Form einer Emulsion vorliegt.

16. Zubereitung nach irgendeinem der vorangehenden Ansprüche, bei welcher es sich um eine topische Zubereitung handelt.

17. Verwendung eines oder mehrerer Extrakte von Soldanella alpina zur Herstellung pharmazeutischer, insbesondere dermatologischer Zubereitungen.

18. Verwendung nach Anspruch 17, bei welcher zusätzlich mindestens ein Extrakt von Saxifraga oppositifolia und/oder Chlamydocapsa sp. eingesetzt wird.

19. Nicht-therapeutische Verwendung von kosmetischen Zubereitungen umfassend (i) ein oder mehrere Extrakte von Saxifraga oppositifolia und/oder (ii) ein oder mehrere Extrakte von Soldanella alpina sowie gegebenenfalls einen oder mehrere Extrakte von Chlamydocapsa sp. zur Verminderung oder Vermeidung von Hautschäden durch extrinsische und/oder intrinsischen Faktoren.

20. Verwendung nach Anspruch 19 **dadurch gekennzeichnet, dass** die Zubereitung mindestens zwei der Extrakte umfasst.

## Claims

1. Cosmetic or dermatological preparation comprising (i) one or more extracts of Saxifraga oppositifolia and/or (ii) one or more extracts of Soldanella alpina.

2. Preparation according to Claim 1, comprising at least (i) one or more extracts of Saxifraga oppositifolia.

3. Preparation according to Claim 1, comprising at least (ii) one or more extracts of Soldanella alpina.

4. Preparation according to Claim 1, comprising both
(i) one or more extracts of Saxifraga oppositifolia and
(ii) one or more extracts of Soldanella alpina.

5. Preparation according to any one of the preceding claims which, based on the total mass of the preparation, comprises not more than 15% by weight, preferably not more than 5% by weight, and in particular from 0.0001 to 0.5% by weight, of in each case (i) and/or (ii).

6. Preparation according to any one of the preceding claims, in which (i) and/or (ii) comprise at least one water-soluble extract.

7. Preparation according to any one of the preceding claims, in which (i) and/or (ii) comprise at least one oil-soluble extract.

8. Preparation according to any one of the preceding claims, in which all extracts (i) and/or (ii) are either water-soluble or oil-soluble.

9. Preparation according to any one of the preceding claims, in which (i) and/or (ii) were obtained by extraction from plant leaves and/or plant stems.

10. Preparation according to any one of the preceding claims which additionally comprises (iii) one or more extracts of Chlamydocapsa sp.

11. Preparation according to any one of the preceding claims which additionally comprises at least one extract different from (i), (ii) and (iii).

12. Preparation according to Claim 11, which additionally comprises one or more extracts selected from the following extracts: Artemisia umbelliformis extract, Rubus idaeus (raspberry) extract, Plantago lanceolata leaf extract, Saccharomyces cerevisiae extract, Panax ginseng root extract, Equisetum arvense extract, Evernia furfuracea (tree moss) extract.

13. Preparation according to any one of preceding Claims 1 to 10, which comprises no additional plant extracts.

14. Preparation according to any one of the preceding claims, which additionally comprises at least one amino acid.

15. Preparation according to any one of the preceding claims, which is present in the form of an emulsion.

16. Preparation according to any one of the preceding claims, in which it is a topical preparation.

17. Use of one or more extracts of Soldanella alpina for producing pharmaceutical, in particular dermatological preparations.

18. Use according to Claim 17, in which additionally at least one extract of Saxifraga oppositifolia and/or Chlamydocapsa sp. is used.

19. Nontherapeutic use of cosmetic preparations comprising (i) one or more extracts of Saxifraga oppositifolia and/or (ii) one or more extracts of Soldanella alpina and optionally one or more extracts of Chlamydocapsa sp. for reducing or avoiding skin damage by extrinsic and/or intrinsic factors.

20. Use according to Claim 19, **characterized in that** the preparation comprises at least two of the extracts.

## Revendications

1. Préparation cosmétique ou dermatologique comprenant (i) un ou plusieurs extraits de Saxifraga oppositifolia et/ou (ii) un ou plusieurs extraits de Soldanella alpina.

2. Préparation selon la revendication 1, comprenant au moins (i) un ou plusieurs extraits de Saxifraga oppositifolia.

3. Préparation selon la revendication 1, comprenant au moins (ii) un ou plusieurs extraits de Soldanella alpina.

4. Préparation selon la revendication 1, comprenant tant (i) un ou plusieurs extraits de Saxifraga oppositifolia qu'également (ii) un ou plusieurs extraits de Soldanella alpina.

5. Préparation selon l'une quelconque des revendications précédentes, qui contient, par rapport à la masse totale de la préparation, pas plus de 15% en poids, de préférence pas plus de 5% en poids et en particulier de 0,0001 à 0,5% en poids à chaque fois de (i) et/ou de (ii).

6. Préparation selon l'une quelconque des revendications précédentes, dans laquelle (i) et/ou (ii) comprend/comprennent au moins un extrait soluble dans l'eau.

7. Préparation selon l'une quelconque des revendications précédentes, dans laquelle (i) et/ou (ii) comprend/comprennent au moins un extrait soluble dans l'huile.

8. Préparation selon l'une quelconque des revendications précédentes, dans laquelle tous les extraits (i) et/ou (ii) sont, soit solubles dans l'eau, soit solubles dans l'huile.

9. Préparation selon l'une quelconque des revendications précédentes, dans laquelle (i) et/ou (ii) a/ont été obtenu(s) par extraction de feuilles et/ou de tiges de plantes.

10. Préparation selon l'une quelconque des revendications précédentes, qui comprend en outre (iii) un ou plusieurs extraits de Chlamydocapsa sp.

11. Préparation selon l'une quelconque des revendications précédentes, qui comprend en outre au moins un extrait différent de (i), (ii) et (iii).

12. Préparation selon la revendication 11, qui comprend en outre un ou plusieurs extraits choisis parmi les extraits suivants : extrait d'Artemisia umbelliformis, extrait de Rubus idaeus (framboise), extrait de feuilles de Plantago lanceolata, extrait de Saccharomyces cerevisiae, extrait de racine de Panax ginseng, extrait d'Equisetum arvense, extrait d'Evernia furfuracea (mousse d'arbre).

13. Préparation selon l'une quelconque des revendications précédentes 1 à 10, qui ne contient pas d'extraits de plantes supplémentaires.

14. Préparation selon l'une quelconque des revendications précédentes, qui contient en outre au moins un acide aminé.

15. Préparation selon l'une quelconque des revendications précédentes, qui se trouve sous forme d'une émulsion.

16. Préparation selon l'une quelconque des revendications précédentes, pour laquelle il s'agit d'une préparation topique.

17. Utilisation d'un ou de plusieurs extraits de Soldanella alpina pour la préparation de préparations pharmaceutiques, en particulier dermatologiques.

18. Utilisation selon la revendication 17, dans laquelle on utilise en outre au moins un extrait de Saxifraga oppositifolia et/ou de Chlamydocapsa sp.

19. Utilisation non thérapeutique de préparations cosmétiques comprenant (i) un ou plusieurs extraits de Saxifraga oppositifolia et/ou (ii) un ou plusieurs extraits de Soldanella alpina ainsi que le cas échéant un ou plusieurs extraits de Chlamydocapsa sp. pour diminuer ou éviter des lésions de la peau par des facteurs extrinsèques et/ou intrinsèques.

20. Utilisation selon la revendication 19, **caractérisée en ce que** la préparation comprend au moins.
deux des extraits.
